**Europäisches Patentamt**

**European Patent Office**

**Office européen des brevets**

(19)

(11) Publication number : **0 457 412 A1**

## (12) EUROPEAN PATENT APPLICATION

(21) Application number : **91201178.0**

(22) Date of filing : **15.05.91**

(51) Int. Cl.⁵ : **G01N 3/42**

(30) Priority : **18.05.90 BE 9000525**

(43) Date of publication of application :
**21.11.91 Bulletin 91/47**

(84) Designated Contracting States :
**BE DE DK FR GB NL**

(71) Applicant : **Wetenschappelijk en Technisch
Centrum van de Belgische Textielnijverheid,
afgekort tot Centexbel
Montoyerstraat 24
B-1040 Brussels (BE)**

(72) Inventor : **Baetens, Ediste Eduard Josephine
Bollewerkstraat 29
B-9031 Drongen (BE)**

(74) Representative : **Debrabandere, René
Bureau De Rycker nv Arenbergstraat 13
B-2000 Antwerpen (BE)**

(54) **Method and device for evaluating the quality of a layer of supple polymer foam.**

(57) Method for evaluating the quality of a layer of supple polymer foam (1), according to which method impression is made in the layer (1) with an impression body (11) and how the layer (1) reacts to this impression is verified, characterised in that with a pre-established constant in time impression in the layer (1) with the assistance of a dynamometer (10) the development of the force on the impression body (11) is registered in function of time and from this development in function of time a characteristic is calculated which depends upon the quality of the layer (1) and of which the relationship with the quality is already known via another method.

*Fig.1*

EP 0 457 412 A1

The invention relates to a method for evaluating the quality of a layer of supple polymer foam, according to which method an impression is made in the layer with an impression body and how the layer reacts to this impression is verified.

A method of this type is utilised for the evaluation of the degree of vulcanisation of a layer of foam latex applied on a substrate, more especially of the foam latex layer applied on the backing of a tufted carpet. This vulcanised foam latex layer is necessary in order to anchor the thread of the wearing layer that is tufted into the base fabric to the backing and in order to give the carpet the necessary resilience against crushing. The layer is applied in the form of a foamed up latex and subsequently vulcanised continuously in an oven through a thermal treatment.

The resilience of the vulcanised foam latex and therefore the quality of the carpet depend upon the measure to which the foam layer is vulcanised. This measure or the degree of vulcanisation is principally dependent upon the temperature and the time the carpet covered with foam remains in the oven. The optimum working conditions however depend upon the composition of the latex paste and can differ from delivery to delivery. The carpet companies must therefore regularly evaluate the degree of vulcanisation of the backing of the carpet.

In fact no real absolute measurement of this degree is performed. It is sufficient to verify whether the degree of vulcanisation is sufficient in order to obtain a desired quality.

A known method for evaluating this degree of vulcanisation consists in that, by means of a stamp, an impression is applied in the vulcanised foam latex layer and it is verified whether, after a certain time, this impression is still visible. If this impression is still visible it is assumed that the foam latex is not sufficiently vulcanised. This primitive method is obviously subjective and hardly reliable.

A more accurate known method consists in measuring the percentage of free sulphur in the vulcanised foam latex by means of chemical analysis. With this method an objective real measurement is obtained but this method is very time consuming and expensive. The result is usually only obtained after a few days so that this method is certainly not suitable for controlling the production of carpet.

The purpose of the invention is to remedy these disadvantages and to provide a method for evaluating the quality of a layer of supple polymer foam, with which an objective evaluation of the quality is possible in a very quick manner, which method is especially suitable for following the production of the layer and which can also be performed on-line.

For this purpose, with a pre-established constant in time impression in the layer, with the assistance of a dynamometer the development of the force on the impression body is registered in function of time and from this development in function of time a characteristic is calculated which depends upon the quality of the layer and of which the relationship with the quality is already known via another method.

In a particular embodiment of the invention a dynamic quantity is calculated as characteristic, based on the mathematical model of a parallel connection of springs and dampers connected in series.

A dynamic response curve is suitably calculated in the frequency field.

In a notable embodiment of the invention complete crushing is effected within a very short time span.

The invention also relates to a device which is particularly suitable for the application of the method according to one of the aforementioned embodiments.

The invention consequently relates to a device for evaluating the quality of a layer of supple polymer foam, which device comprises an impression body and of which the characteristic consist in that it comprises means for impressing the impression body to a pre-established degree in the polymer foam, a dynamometer which is mounted on the impression body in order to measure the force exerted by the polymer foam on the body for a specified time and a computer which on the one hand ensures the control of the movement of the dynamometer along a vertical axis and which on the other hand is connected to the dynamometer in order in function of the signals originating from the dynamometer corresponding to the development of the force in function of time to calculate a characteristic which depends upon the quality of the layer.

In a particular embodiment the means for impressing the impression body in the polymer foam comprise a stepping motor.

In a notable embodiment of the invention the contact surface of the impression body with the layer is a curved surface.

In order to show better the characteristics according to the present invention, some preferred embodiments of a method and a device for evaluating the quality of a layer of supple polymer foam, according to the invention, are described hereafter, as examples and without any restrictive character, with reference to the enclosed drawings, in which:

Figure 1 shows in schematic manner a device for evaluating the quality of the foam latex layer of a tufted carpet according to the invention;

figure 2 shows a section according to line II-II, but drawn to larger scale;

figure 3 shows the development of the force in function of time during the measurement;

figure 4 shows the development of the loss factor in function of the logarithm of the frequency calculated from the aforementioned development.

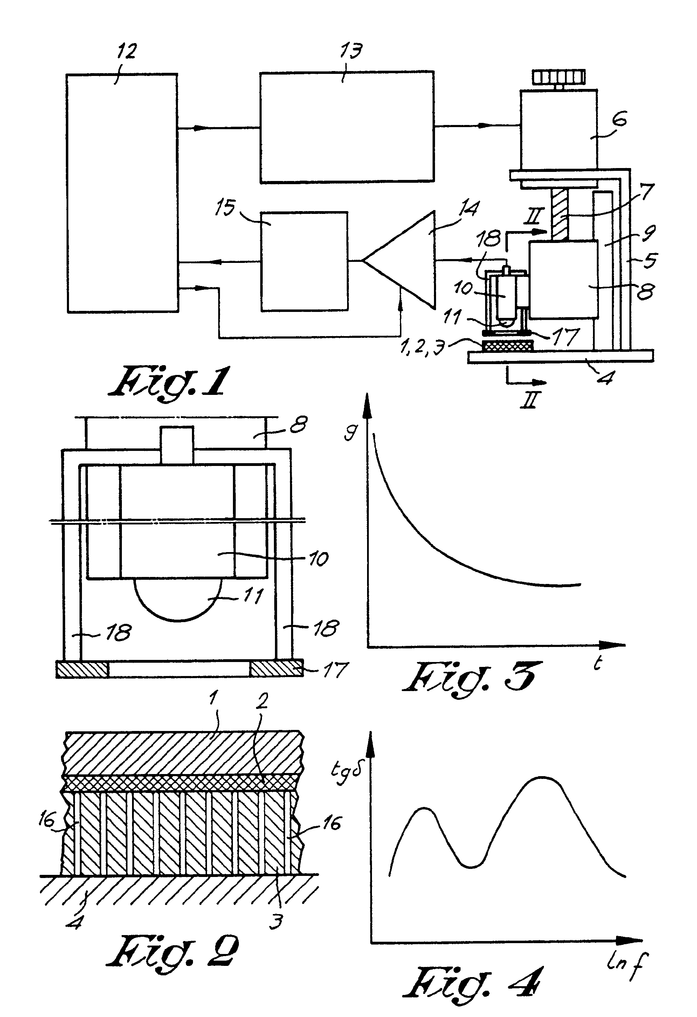

Figure 1 shows a device for the off-line evaluation of the degree of vulcanisation of the foam latex backing 1 of a tufted carpet. This foam backing 1 is applied to a base fabric 2 to which the wearing layer 3 is applied by tufting.

This device comprises a horizontal table 4 on which a piece of the carpet 1, 2, 3 is placed. A stepping motor 6 is mounted on a vertical support 5 above the table 4. The downward directed shaft 7 of the motor 6 is provided with screw thread and with rotation moves a table 8 mounted thereon which is movable upward and downward over a guide 9 mounted on the support 5.

On one side the table 8 bears a dynamometer 10 for the measurement of the counter pressure of the piece of carpet 1, 2, 3. The dynamometer 10 bears an impression body 11 underneath of which the under side forms a part of a curved surface, and of which the dimensions are large in relation to the microstructure of the layer to be measured.

A central computer 12 with a very stable clock frequency controls the stepping motor 6 over the interface 13. The signals from the dynamometer 10 are sent through an amplifier 14, with an amplification controlled by the computer 12, and an analogue-digital converter 15 to the computer 12.

The sample, this is the piece of carpet 1, 2, 3 is placed on the table 4 with the wearing layer 3 underneath. In order to exclude the influence of the wearing layer on the measurement the base fabric 2 and therefore the latex layer 1 is supported by a number of needles 16 which are on the table 4 and extend through the wearing layer 3 against the base fabric 2. A good contact of the layer 1 with the needles 16 can possibly be ensured by a pressing body 17, 18 that is applied to the fabric 1, 2, 3 together with a dynamometer 10 through the table 8. As is shown in detail in figure 2, this pressing body 17, 18 consists of a horizontal ring 17 and arms 18 with which the ring 17 is suspended loosely from this dynamometer in the highest position of the dynamometer 10 shown in figure 2.

In order to evaluate the degree of vulcanisation of the latex foam backing 1 of the sample the following is undertaken:

Through intervention of the computer 12 the stepping motor 6 is started until this motor 6 has moved the unit formed by the table 8, the dynamometer 10 the impression body 11 and, if a pressing body 17, 18 is present, also this pressing body so far downward that the impression body is in contact with the piece of carpet 1, 2, 3 and is therefore in its starting position for a measurement. The latter is necessary because the further impression with the impression body 11 must occur very quickly, for example within a time span of 50 ms. If a pressing body 17, 18 is present, before the dynamometer 10 has reached the aforementioned starting position, this pressing body 17, 18 comes to rest with its ring 17 on the piece of carpet 1, 2, 3. The dynamometer 10 can thereafter, free from the pressing body 10, further descend until the impression body 11 within the ring 17 comes into contact with the piece of carpet 1, 2, 3. This previous movement of the impression body 11 can be determined in two ways. The movement can take place over a fixed, previously determined distance which is determined by the stepping motor 6 and among others the number of pulses which the computer 12 transmits through the interface 13 to this stepping motor 6. The movement can also occur until the dynamometer 12 registers a well defined relatively small counter pressure from the layer 1, after which the computer 12 causes the stepping motor to stop.

Subsequently the computer starts the actual measurement. In doing so it commands the stepping motor 6 to move the impression body 11 linearly downward over an adjustable distance which lies between 10% and 30% of the thickness of the layer. As already mentioned this movement occurs very quickly.

In theory the penetration occurs immediately and data collection can start through the dynamometer 10, this is therefore the moment that this dynamometer transmits signals to the computer 12, at the moment that the stepping motor 6 receives the command to penetrate the foam latex of the layer 1. In practice the data collection starts however at the time that the penetration is finished. Also because of this it is necessary that this impression body 11 is positioned just above the surface of the layer 1 or slightly in the layer 1 with the aforementioned previous movement before this command is given.

An immediate deformation according to an ideal step is for all sorts of reasons, such as mechanical inertia and material resistance not possible. This means that a specific time is necessary before the impression body 11 has penetrated to the desired depth in the layer 1. If the actual deformation occurs very quickly in relation to the dynamic response of the material in time, this deformation can approximately be considered equal to the theoretical immediate deformation and further calculation is effected correctly. If conversely the deformation does not occur sufficiently quickly then the deformation development during the impression of the impression body 11 will have to be taken into account.

The dynamometer 10 transmits a signal corresponding to the measured force to the computer 12 at regular small intervals, for example every 10 ms. The dynamic model that corresponds to a viscoelastic material such as foam latex now predicts a curve of the force in function of time which for the first moments after the maximum impression of the impression body 11 decreases very quickly and thereafter very slowly dies out over a long period. The curve which this development shows is shown in figure 3. Consequently an equidistant distribution of the positive points over the entire time span is not necessary or desirable for the mathematical processing. In a particular embodiment of the method the logarithmic selection of the positive points can be retained for further mathematical processing.

From this curve, also called declining curve or relaxation decline, subsequently dynamic quantities and namely the dynamic modulus of elasticity, the dynamic loss modulus and the loss factor are calculated by the computer 12, based on a mathematical model consisting of a parallel connection of springs and dampers connected in series.

Springs follow Hooke's law namely f=E x e whereby e is the enforced deformation, f the tension created and E the modulus of elasticity. This law is valid for a perfectly elastic material that after deformation regains its original condition.

Dampers follow Newton's law, namely: f=n(de/dt) whereby n is the coefficient of viscosity. The derivative of e to t is the speed of the deformation.

If the deformation e applied is constant, as is the case with the present method, then in an ideal elastic material this will cause a tension which is proportional to the deformation applied but cause no tension in an ideal viscous material which signifies that the material is permanently deformed.

In order to come to precise mathematical analogies with specific viscoelastic materials, such as the vulcanised foam latex considered here, complex combinations of ideal springs and ideal dampers are necessary.

A model, consisting of a combination of a spring and a damper permits an interpretation to be given to an experimentally observed behaviour that is characterised by a tension decreasing in time with applied constant deformation. Each combination of a spring and a damper in series gives a tension f(i), which is a solution of the indicated basic equation.

A parallel connection of such combinations gives an equation of which the solution is given by the sum of the individual tensions f(i). If an infinite parallel connection of the combinations "spring and damper in series" is used as model for the viscoelastic behaviour of vulcanised foam latex then the development of the force which arises as response for the material at a constant deformation, in function of time, as presented in figured 3, can be described by an infinite sum of exponentials:

$$f(t) = SUM (f(i)) = SUM (a(i) \exp (-t/T(i)))$$

Hereby a(i) is a pre-exponential factor (amplitude) and T(i) a time constant. The different T(i) are dependent upon the foam latex and therefore material constants if the mathematical model is a good description of the true viscoelastic behavior of the foam latex.

The parameters a(i) and T(i) are calculated by a non-linear smallest squares method from the development of the force in function of time.

Although the response curve as shown in figure 3 can perfectly described by an infinite sum of exponentials, it is sufficient for the practical embodiment to limit the series to an infinite number of terms.

Such finite series of exponentials are brought into line with the experimental measurements. From these parameters a(i) and T(i) the frequency response of the foam latex layer 1 can be calculated.

The frequency response expected according to the model of the material to an applied harmonic deformation with pulsation w gives a tension response which has the same pulsation, but which is out of phase in relation to the applied deformation. The three aforementioned dynamic quantities: dynamic modulus of elasticity E', dynamic loss modulus E" and the loss factor tangent delta can be calculated from the aforementioned parameters T(i) and a(i).

The dynamic frequency response on the basis of the afore mentioned theoretical model is given by an harmonic function with amplitude $(E'2 + E"2)^{\frac{1}{2}}$ and a dephasing delta.

In this manner, taking the development of the force in function of time (fig. 3) the computer 12 calculates the development of the loss factor (the tangent of the dephasing delta) in function of the natural logarithm of the frequency, which possible development is shown in figure 4.

For each impression the latter development is compared to a table or graph which establishes the correlation between these values and the degree of vulcanisation as determined by the use of the chemical analysis technique. In order to obtain this correlation table or graph reference samples with varying degrees of vulcanisation are on the one hand analysed according to the chemical method and on the other hand according to the above described method according to the invention. Once this correlation table or graft is established, this can be used for all measurements which are conducted according to the invention on similar samples with unknown degree of vulcanisation. For this comparison various criteria can be applied, for example the decline characteristics from the force response as shown in figure 3, or the form and surface area characteristics from the development of the loss factor.

In a variant of the above described method, subject to an adjustment of the measurement device, the degree of vulcanisation is measured on-line. The latexing line always comprises a buffer and the measurement, more specifically the impression with the impression body 11, can therefore occur on stationary carpet.

The table 4 is under the wearing layer 3. The measuring head formed by the motor 6, the threated shaft 7, the table 8, the guide 9, the dynamometer 10 and the impression body 11, is mounted above the foam latex layer 1. With the exception of this position of the measuring head and the table 4, the method is in fact identical to the above described method.

The measuring head can be movably disposed in the transverse direction of the latexing line whereby, still with stationary carpet, the degree of vulcanisation can be evaluated at various places in the width direction of the foam latex layer 1.

In a still further variant the method is applied to a moving carpet. In this case a measuring head and a table 4 are used which move along with the carpet during the application of the method. The impression body 11 is stationary in relation to the layer 1 during the application.

The above described method permits a fast evaluation of the degree of vulcanisation which is furthermore objective so that the evaluation can be used for the control of the device for the application and vulcanisation of the foam latex layer 1.

The present invention is in no way restricted to the embodiments described as examples and shown in the drawings, but such method and device for evaluating the quality of a layer of supple polymer foam may be developed in different variants without departing from the scope of the present invention.

In particular the invention is not limited to the evaluation of the degree of vulcanisation of a layer of foam latex of a carpet. The invention is also applicable to foam latex layers on other substrates than carpet and even on other supple polymer foam than latex. In the last case the degree of vulcanisation is not evaluated but for example other qualities such as the net formation with polyurethane foam.

## Claims

1.- Method for evaluating the quality of a layer of supple polymer foam (1), according to which method impression is made in the layer (1) with an impression body (11) and how the layer (1) reacts to this impression is verified, characterised in that with a pre-established constant in time impression in the layer (1) with the assistance of a dynamometer (10) the development of the force on the impression body (11) is registered in function of time and from this development in function of time a characteristic is calculated which depends upon the quality of the layer (1) and of which the relationship with the quality is already known via another method.

2.- Method according to the preceding claim, characterised in that based on the mathematical model of a parallel connection of springs and dampers connected in series as characteristic a dynamic quantity is calculated.

3.- Method according to the preceding claim, characterised in that a dynamic response curve is calculated in the frequency field.

4.- Method according to the preceding claim, characterised in that the development of the loss factor is calculated in function of the frequency.

5.- Method according to the preceding claim, characterised in that based on the mathematical model of a parallel connection of springs and dampers connected in series the measured development of the force on the dynamometer (10) in function of time is made to correspond with a mathematical equation which shows the connection between force and time, the most suitable parameters a(i) and T(i) which the equation comprises are calculated and subsequently from these parameters the development of the loss factor is calculated in function of the frequency.

6.- Method according to any of the preceding claims, characterised in that the layer (1) is held stationary during the impression.

7.- Method according to any of the preceding claims, characterised in that complete impression is effected within a very short time span of for example 50 ms.

8.- Method according to any of the preceding claims, characterised in that the impression body (11) is pressed in the layer (1) over an adjustable depth which is included between 10% and 30% of the thickness of the layer.

9.- Method according to any of the preceding claims, characterised in that the impression body (11) is first brought into the vicinity of the surface of the layer (1) and only then is the impression performed whereby the force is registered by means of the dynamometer (10).

10.- Method according to any of the preceding claims, characterised in that an impression is made in a foam layer (1) on a substrate (2, 3) with a base fabric (2) and a wearing layer (3) and the base fabric (2) is supported in order to exclude influence through deformation of the wearing layer (3) during the impression in the layer (1).

11.- Method according to the preceding claim, characterised in that during the impression not only the substrate (2, 3) is supported by a support (16) but is also pressed against this support.

12.- Method according to any of the preceding claims, characterised in that the degree of vulcanisation is evaluated of the foam latex layer (1) of a carpet.

13.- Method according to the claims 5 and 12, characterised in that the development of the loss factor is calculated in function of the frequency and this development is compared to similar developments of which the degree of vulcanisation was evaluated by another method.

14.- Device for evaluating the quality of a layer of supple polymer foam (1), which device comprises an impression body (11), characterised in that it comprises means (6) for impressing the impression body (11) to a pre-established degree in the polymer foam (1), a dynamometer (10) which is mounted on the impression body (11) in order to measure the force exerted by the polymer foam (1) on the impression body (11) for a specified time and a computer (12) which is connected to the dynamometer (10) in ruder in function of the signals originating from the dynamometer (10) corresponding to the development of the force in function of time to calculate a characteristic which depends upon the layer (1).

15.- Device according to the preceding claim, characterised in that the means (6) for pressing the impression body (11) in the polymer foam layer comprise a stepping motor (6).

16.- Device according to either of the claims 14 and 15, characterised in that the contact surface of the impression body (11) with the layer (1) is a curved surface.

17.- Device according to either of the claims 14 to 16, characterised in that it comprises a support (16) for the layer (1).

18.- Device according to the preceding claim, characterised in that the layer (1) forms the backing of a carpet (1, 2, 3) and the support (16) comprises needles which reach through the wearing layer (3) of the carpet to on or in the base fabric (2) on which the wearing layer (3) is applied.

19.- Device according to either of the claims 17 and 18, characterise d in that it comprises an impression body (17, 18) that is mounted movably up and down above the support (16).

20.- Device according to the preceding claim, characterised in that the pressing body (17, 18) is loosely suspended from the dynamometer (10).

21.- Device according to the preceding claim, characterised in that the pressing body (17, 18) comprises a ring (17) and at least one suspension element (18) with which it loosely hangs from the dynamometer (10) when this is in its highest position.

*Fig.1*

*Fig.2*

*Fig. 3*

*Fig. 4*

European Patent
Office

**EUROPEAN SEARCH REPORT**

Application Number

EP 91 20 1178

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 140 008 (G.A. GOLEMBECK et al.) * Column 1, line 65 - column 3, line 49; figures 1-5 * | 1,14 | G 01 N 3/42 |
| A | US-A-4 450 713 (T. ARIMATSU) * Column 2, line 11 - column 4, line 25; figures 1-10 * | 1,14 | |
| A | GR-A-2 595 824 (INSTITUT NATIONAL DE LA RECHERCHE AGRONOMIQUE) * Page 5, line 4 - page 8, line 18; figures 1-3 * | 1,14 | |
| | | | TECHNICAL FIELDS SEARCHED (Int. Cl.5) |
| | | | G 01 N 3/00 G 01 N 33/00 |

The present search report has been drawn up for all claims

| Place of search | Date of completion of the search | Examiner |
|---|---|---|
| THE HAGUE | 26-06-1991 | SARNEEL A.P.T. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)